(19) Europäisches Patentamt / European Patent Office / Office européen des brevets

(11) **EP 4 656 227 A1**

(12) **EUROPEAN PATENT APPLICATION**
published in accordance with Art. 153(4) EPC

(43) Date of publication:
**03.12.2025 Bulletin 2025/49**

(21) Application number: **24763749.9**

(22) Date of filing: **21.02.2024**

(51) International Patent Classification (IPC):
**A61M 21/02** (2006.01)    **A61B 5/16** (2006.01)

(52) Cooperative Patent Classification (CPC):
**A61B 5/16; A61M 21/02**

(86) International application number:
**PCT/JP2024/006217**

(87) International publication number:
**WO 2024/181268 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.02.2023 JP 2023030309**

(71) Applicant: **JVCKenwood Corporation
Yokohama-shi, Kanagawa 2210022 (JP)**

(72) Inventor: **SUZUKI, Ako
Yokohama-shi, Kanagawa 221-0022 (JP)**

(74) Representative: **Pritzlaff, Stefanie Lydia
Wagner & Geyer Partnerschaft mbB
Patent- und Rechtsanwälte
Gewürzmühlstraße 5
80538 München (DE)**

(54) **INDUCTION DEVICE, INDUCTION METHOD, AND PROGRAM**

(57)    A subject can be guided to have an appropriate dream. A guidance device includes a biological information acquiring unit (21) configured to acquire biological information of a subject, a memory (13, 13A) configured to store therein multiple scenarios created using multiple pieces of image data associated to each other, and a selecting unit (24, 24A) configured to select, from among the multiple scenarios stored in the memory (13, 13A), the scenario related to the biological information acquired by the biological information acquiring unit (21).

FIG.1

## Description

### Field

The present disclosure relates to a guidance device, a guidance method, and a program.

### Background

In recent years, there is advancement in the technology for measuring brain activity information, and the technology about a brain-machine interface, which represents the interface between the brain and the outside, is becoming more and more realistic. In Patent Literature 1 mentioned below, it is disclosed that the variation in the depth of sleep of the subject, who is asleep, is predicted, and if there is a transitional state in which the depth of sleep of the subject increases, a stimulation is applied to the subject before the arrival of the waking time.

### Citation List

### Patent Literature

[Patent Literature 1] Japanese Patent No. 5958553

### Summary

However, in Patent Literature 1, there is no suggestion about guiding the subject, who is in a sleep state, to have a dream.

The present disclosure is made in view of the issues mentioned above, and it is an objective of the present disclosure to provide a guidance device, a guidance method, and a program that can guide the subject to have an appropriate dream.

According to one aspect of the present disclosure, there is provided a guidance device comprising: a biological information acquiring unit configured to acquire biological information of a subject; a memory configured to store therein multiple scenarios created using multiple pieces of image data associated to each other; and a selecting unit configured to select, from among the multiple scenarios stored in the memory, the scenario related to the biological information acquired by the biological information acquiring unit.

According to one aspect of the present disclosure, there is provided a guidance method comprising: acquiring biological information of a subject; storing multiple scenarios created using multiple pieces of image data associated to each other; and selecting, from among the stored multiple scenarios, the scenario related to the acquired biological information.

According to one aspect of the present disclosure, there is provided a program that causes a computer, which operates as a guidance device, to execute: acquiring biological information of a subject; storing multiple scenarios created using multiple pieces of image data associated to each other; and selecting, from among the stored multiple scenarios, the scenario related to the acquired biological information.

According to the present disclosure, the subject is guided to have an appropriate dream.

### Brief Description of Drawings

FIG. 1 is a block configuration diagram illustrating a guidance device according to a first embodiment;
FIG. 2 is a graph for explaining physiological characteristics of a biological signal;
FIG. 3 is a schematic diagram for explaining the autonomic nervous system activity.
FIG. 4 is a flowchart for explaining a guidance method according to the first embodiment;
FIG. 5 is a block configuration diagram illustrating a guidance device according to a second embodiment; and
FIG. 6 is a flowchart for explaining a guidance method according to the second embodiment.

### Description of Embodiments

Exemplary embodiments of a guidance device, a guidance method, and a program according to the present disclosure are described below in detail with reference to the accompanying drawings. However, the present invention is not limited by the embodiments described below.

[First embodiment]

<Guidance device>

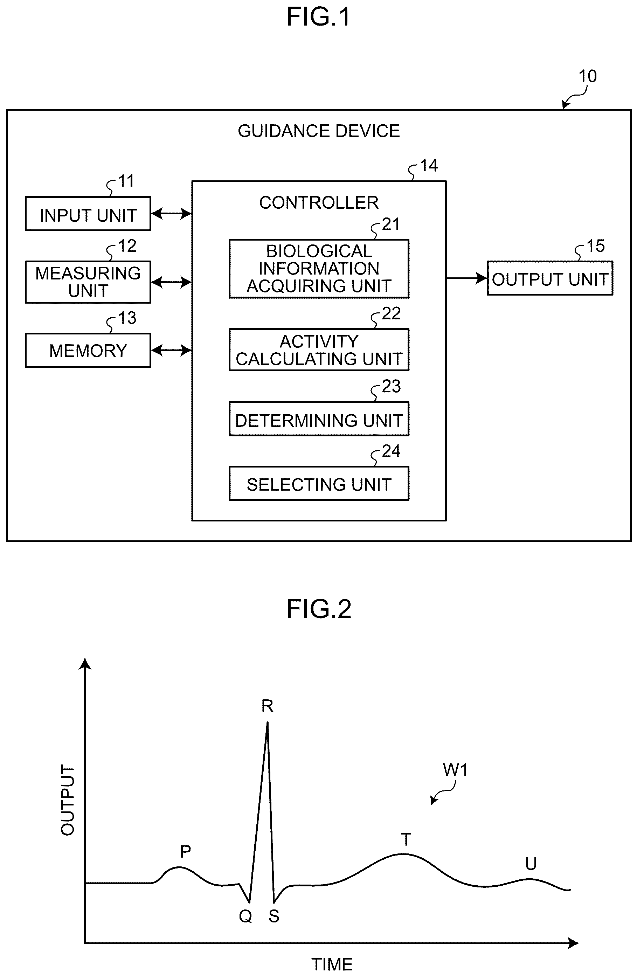

FIG. 1 is a block configuration diagram illustrating a guidance device according to a first embodiment.

As illustrated in FIG. 1, a guidance device 10 guides a user (subject), who is in a sleep state, to have an appropriate dream. The guidance device 10 includes an input unit 11, a measuring unit 12, a memory 13, a controller 14, and an output unit 15.

The input unit 11 is connected to the controller 14. The input unit 11 is operable by the user, and is capable of inputting various signals to the controller 14. For example, the input unit 11 inputs, to the controller 14, a start signal for starting a guidance to enable the user to have a dream or an end signal for ending the guidance to the user. The input unit 11 can be implemented using, for example, a touch-sensitive panel, buttons, switches, or a keyboard.

The user can operate the input unit 11 and create a scenario. Regarding a scenario, the detailed explanation is given later. Moreover, the user can operate the input unit 11 and input a category of the dream to be induced. Herein, the category indicates a phenomenon related to a hobby, a phenomenon related to the family, a phenomenon related to the work, or a phenomenon related to world events. Moreover, the category indicates an enjoyable phenomenon, a sad phenomenon, a delightful phenomenon, or an adventurous phenomenon. Thus, the user can operate the input unit 11 and select the type of the dream to be induced.

The measuring unit 12 is connected to the controller 14. Based on a program, the controller 14 provides a measurement signal to the measuring unit 12. Then, based on the measurement signal input from the controller 14, the measuring unit 12 measures biological information of the user.

The measuring unit 12 is a biological sensor that detects the biological information of the user. As long as the biological information of the user can be detected, the biological sensor can be installed at an arbitrary position. Herein, the biological information does not imply permanent information such as the fingerprints, but implies values that vary according to a condition of the user. That is, the biological information represents information related to the autonomic nerves of the user, that is, information that changes in values regardless of an intention of the user.

As the biological information, the measuring unit 12 measures, for example, the brain waves, the cerebral blood value, the heart rate, the respiratory rate, the blood pressure, the body temperature, the amount of perspiration, and the myoelectric current. As the measuring unit 12, for example, a measurement device that performs measurement based on a principle of fMRI (which stands for functional Magnetic Resonance Imaging) or fNIRS (which stands for functional Near-Infrared Spectroscopy), a measurement device in which an invasive electrode is used, or a measurement device that performs measurement using micromachines that are placed inside blood vessels of the brain is able to be used.

Alternatively, the measuring unit 12 can be a pulse wave sensor as the biological sensor. Accordingly, the measuring unit 12 detects pulse waves of the user as the biological information. For example, the pulse wave sensor can be a through-beam photoelectric sensor that includes a light emitting unit and a light receiving unit. In that case, for example, the pulse wave sensor is configured in such a way that the light emitting unit and the light receiving unit face each other across the fingertip of the user, the light receiving unit receives light which has passed through the fingertip, and a pulse waveform is measured based on the fact that the blood flow is higher in proportion to the pressure of the pulse waves. However, the pulse wave sensor is not limited to have the configuration explained above, and can be configured in an arbitrary manner as long as the pulse waves can be detected.

The memory 13 is connected to the controller 14. The memory 13 stores therein a variety of information. In the memory 13, an activity threshold value which is used during a guidance operation performed by the controller 14 is stored in advance. The activity threshold value is, for example, a preset threshold value of an autonomic nervous system activity, and represents degree of clarity of phenomenon in a dream of the user who has a dream in a sleep state. From among dreams, those dreams which are story-centered and which can be recalled in detail after waking up are often observed during REM sleep. On the other hand, from among dreams, those dreams which are not story-centered and which are fragmentary are often observed in non-REM sleep. During REM sleep, there is an increase in the autonomic nervous system activity (explained later), and during non-REM sleep, there is a decrease in the autonomic nervous system activity. For that reason, the autonomic nervous system activity serves as a guideline for indicating degree of clarity of a phenomenon in a dream of the user who has a dream in the sleep state. An emotional level threshold value is, for example, a preset threshold vale of emotional level, and represents degree of variation in emotion toward the phenomenon in the dream of the user who has a dream in the sleep state.

Moreover, scenarios are also stored the memory 13. A scenario is created by chronologically arranging multiple pieces of image data associated to each other. More particularly, a scenario is created by chronologically arranging multiple pieces of image data corresponding to multiple specific phenomena related to each other. For example, when an airplane represents a specific first phenomenon, the sky represents a second phenomenon related to the airplane representing the first phenomenon; the clouds represent a third phenomenon related to the sky representing the second phenomenon; the thunderstorm represents a fourth phenomenon related to the clouds representing the third phenomenon; the lightning strike represents a fifth phenomenon related to the thunderstorm representing the fourth phenomenon; and the fire represents a sixth phenomenon related to the lightning strike representing the fifth phenomenon. Thus, a scenario about an airplane is created by chronologically arranging the image data of the first phenomenon (airplane), the image data of the second phenomenon (sky), the image data of the third phenomenon (clouds), the image data of the fourth phenomenon (thunderstorm), the image data of the fifth phenomenon (lightning strike), and the image data of the sixth phenomenon (fire).

In that case, a scenario about an airplane is not limited to the scenario explained above. Alternatively, for example, travelling represents the second phenomenon related to the airplane representing the first phenomenon; a foreign country represents the third phenomenon related to the travel representing the second phenomenon; Europe represents the fourth phenomenon related to the foreign country representing the third phenomenon; Paris represents the fifth phenomenon related to Europe representing the fourth phenomenon; and the Arc de Triomphe represents the sixth phenomenon related to Paris representing the fifth phenomenon. Thus, the scenario of an airplane is created by chronologically arranging the image data of the first phenomenon (airplane), the image data of the second phenomenon (travelling), the image data of the third phenomenon (foreign country), the image data of the fourth phenomenon (Europe), the image data of the fifth phenomenon (Paris), and the image data of the sixth phenomenon (Arc de Triomphe). Thus, multiple scenarios about an airplane are stored in the memory 13.

Meanwhile, in one scenario, the number of chronologically-arranged pieces of image data is not limited to six, but can be equal to or lower than five or equal to or greater than seven. Moreover, a type of the first phenomenon is not limited to an airplane, and a large number of other phenomena can represent the first phenomenon. Thus, a large number of scenarios are stored in the memory 13.

Herein, scenarios are created by the users by operating the input unit 11. Thus, multiple scenarios corresponding to hobbies and experiences of specific users are created and stored in the memory 13. Alternatively, multiple scenarios corresponding to the hobbies of typical users can be created and stored in the memory 13.

Moreover, in the memory 13, a program is stored that enables the controller 14 to provide guidance. The memory 13 is an external storage device such as an HDD (which stands for Hard Disk Drive), or is a memory.

The controller 14 includes a biological information acquiring unit 21, an activity calculating unit 22, a determining unit 23, and a selecting unit 24. For example, the controller 14 is configured using an arithmetic circuit such as a CPU (which stands for Central Processing Unit).

The biological information acquiring unit 21 is connected to the measuring unit 12. The biological information acquiring unit 21 controls the measuring unit 12 and causes the measuring unit 12 to detect the biological information of the measuring unit 12. Then, the biological information acquiring unit 21 acquires the biological information of the user measured by the measuring unit 12.

The biological information acquiring unit 21 is connected to the activity calculating unit 22. The activity calculating unit 22 calculates the autonomic nervous system activity based on a biological signal acquired by the biological information acquiring unit 21. Regarding a calculation method implemented by the activity calculating unit 22 to calculate the autonomic nervous system activity, the explanation is given later.

The activity calculating unit 22 is connected to the memory 13 and the determining unit 23. The determining unit 23 compares the autonomic nervous system activity, which is calculated by the activity calculating unit 22, with an activity threshold value stored in the memory 13, and determines whether or not the guidance operation can be performed. More particularly, when the user is, in the sleep state, having a dream and recalling a specific phenomenon (sensory information), the determining unit 23 compares the autonomic nervous system activity of the user at that time with the activity threshold value, and accordingly determines whether or not the guidance operation is to be performed. When the autonomic nervous system activity of the user is higher than the activity threshold value, that is, when the user in the sleep state is having the high degree of clarity of a phenomenon in a dream of the user, the determining unit 23 determines that the guidance operation is to be performed. On the other hand, when the autonomic nervous system activity of the user is equal to or lower than the activity threshold value, that is, when the user in the sleep state is having the low degree of clarity of a phenomenon in a dream of the user, the determining unit 23 determines that the guidance operation is not to be performed. It is desirable that the activity threshold value is set on a user-by-user basis. In that case, in the sleep state of the user, multiple relationships between the degree of clarity of the recalled phenomenon and the autonomic nervous system activity are obtained in advance. Then, in the obtained multiple relationships between the degree of clarity of the recalled phenomenon and the autonomic nervous system activity, it is desirable to set the activity threshold value according to the degree of clarity of a phenomenon which the user wishes to be guided in a dream by the guidance operation for having a dream.

The determining unit 23 is connected to the memory 13 and the selecting unit 24. Moreover, the selecting unit 24 is connected to the biological information acquiring unit 21, the determining unit 23, and the memory 13. The selecting unit 24 selects, from the multiple scenarios stored in the memory 13, the scenario related to the biological information of the user which is acquired by the biological information acquiring unit 21. That is, when the determining unit 23 determines that the autonomic nervous system activity calculated based on the biological information of the user is higher than the activity threshold value, the selecting unit 24 selects the scenario related to the phenomenon in the dream of the user in the sleep state.

As a method for estimating the phenomenon in the dream of the user in the sleep state, the following technology is known. For example, a sparse coding theory, which is a method for visualization of transition of recognition from the first visual cortex, is implemented in which, an FMRI activity map of the visual cortex is visualized by a DNN (Deep Neural Network)-CNN (Convolutional Neural Network), simply and locally processed in the primary visual cortex, and then recognized in a stepwise manner in the secondary visual cortex. According to this method, in the brain stimulation and the brain cognition (cognition of having a dream in case of a vision) that is unique to the user, the relationship among the trigger, the recalled image, and the sound, that is, the image data in the brain recalled by the trigger can be obtained according to the biological information of the user.

Then, image processing such as pattern matching is performed with respect to the obtained image data of the user to estimate the phenomenon in the dream of the user, and a scenario is selected that is related to the phenomenon in the dream of the user in the sleep state. In that case, for example, it is possible to implement a method for similar-image retrieval. The similar-image retrieval includes: a classification operation for classifying the contents (for example, an airplane) of the image data; an object detection operation for finding objects in the image data; a face recognition operation for recognizing faces from the image data to determine the age, the gender, and identicalness of the persons; and a retrieval operation for retrieving an image that is similar to the specified image. In the first embodiment, the classification operation and the object detection operation are performed to detect related objects (for example, an airplane, a train, and an automobile) from the image data corresponding to the biological information of the user. Then, a scenario is selected that is related to the phenomenon of the object (for example, the airplane) which, from among the retrieved objects, is determined to have the autonomic nervous system activity higher than the activity threshold value.

The controller 14 is connected to the output unit 15. Based on a result of control performed by the controller 14, that is, based on the scenario selected by the selecting unit 24, the output unit 15 stimulates the brain of the user. Herein, the output unit 15 stimulates the brain of the user in such a way that multiple pieces of related image data set in the selected scenario is replayed at regular time intervals (for example, intervals of one minute). In that case, since the replayed pieces of image data are associated to each other, the user becomes able to recall the specific scenario. In that case, along with replaying the related pieces of image data, preset sound data can be replayed in accordance with the phenomenon in the image data.

Regarding the replay of images in the brain by stimulating the brain of the user, for example, it becomes necessary to perform machine learning in advance and to understand a relationship among degree of applied stimulation (for example, stimulation using electromagnetic waves or using an electrode needle) as a teacher data set, portion of brain cells of the user in which the stimulation is applied, and degree of accuracy of the scenario recalled by the user based on the applied stimulation.

<Autonomic nervous system activity>

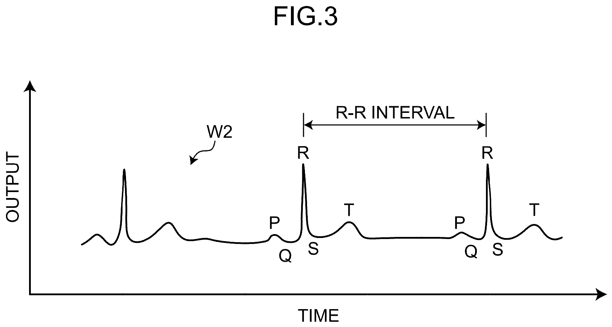

FIG. 2 is a graph for explaining physiological characteristics of a biological signal. FIG. 3 is a schematic diagram for explaining the autonomic nervous system activity. In the explanation of FIGS. 2 and 3, the biological signal is assumed to be a pulse wave signal related to a pulse wave. However, instead of a pulse wave, the biological signal such as a brain wave can be used.

As illustrated in FIG. 2, a waveform W1 representing a pulse wave signal includes a P wave, a QRS wave, a T wave, and a U wave. The heart rate variability is measured by detecting the R wave which represents a peak of the QRS wave as one pulse.

The pulse wave is a waveform in which peaks called R-wave WR appear at regular time intervals. The pulse occurs due to autoignition of pacemaker cells in the sinoatrial node of the heart. Rhythm of the pulse is heavily influenced by the sympathetic nervous system and the parasympathetic nervous system. The sympathetic nervous system enhances the heart activity, while the parasympathetic nervous system suppresses the heart activity. Normally, the sympathetic nervous system and the parasympathetic nervous system act to counterbalance each other. When at rest or in a state close to resting, the parasympathetic nervous system becomes dominant. Normally, when adrenaline is secreted due to the activation of the sympathetic nervous system, the pulse rate increases. On the other hand, when acetylcholine is secreted due to the activation of the parasympathetic nervous system, the pulse rate decreases. Hence, regarding a functional inspection of the autonomic nerve system, it is assumed that checking variability in an R-R interval in the electrocardiogram proves useful.

As illustrated in FIG. 3, in a waveform W2 representing the pulse wave signal, the R-R interval indicates an interval between chronologically continuous R-wave WR. The heart rate variability is measured by treating the R wave, which represents the peak of the QRS wave, as one pulse. The variability in the interval between the R waves in the electrocardiogram, that is, a fluctuation in the time interval of the R-R interval in FIG. 3 is used as an autonomic nervous system indicator. An appropriateness of using the fluctuation in the time interval of the R-R interval as the autonomic nervous system indicator has been reported in many medical institutions. The fluctuation of the R-R interval increases when at rest and decreases when in stress.

The variability in the R-R interval includes a few types of characteristic fluctuations. One type of fluctuation represents low-frequency component appearing in the vicinity of 1 Hz and is attributed to the variation in the sympathetic nervous system along with the blood pressure feedback control of the blood vessels. Another type of fluctuation indicates the variation occurring in synchronization with breathing, and represents high-frequency component that reflect the respiratory sinus arrhythmia. The high-frequency component reflects direct interference with vagal preganglionic neuron due to the respiratory center, stretch receptor reflex, and baroreceptor reflex of the blood pressure change due to the breathing, and is treated as the parasympathetic nervous system indicator that mainly affects the heart. That is, it can be said that, from among waveform components in which the fluctuation between the R-R waves of the pulse wave is measured, power spectrum of the low-frequency component represents the activity of the sympathetic nervous system, and power spectrum of the high-frequency components represent the activity of the parasympathetic nervous system.

The fluctuation of the input pulse wave is obtained from a differential value of the R-R interval value. In that case, when the differential values of the R-R intervals do not represent equally spaced time-series data, the activity calculating unit 22 converts those values into equally spaced time-series data using a three-dimensional spline interpolation. The activity calculating unit 22 performs orthogonal transform such as fast Fourier transform with respect to the differential values of the R-R intervals. Thus, the activity calculating unit 22 calculates the power spectrum of the high-frequency components and the power spectrum of the low-frequency components of the differential values of the R-R interval values of the pulse wave. The activity calculating unit 22 calculates a sum total of the power spectrum of the high-frequency component as RRHF. Moreover, the activity calculating unit 22 calculates a sum total of the power spectrum of the low-frequency component as RRLF. The activity calculating unit 22 calculates the autonomic nervous system activity using the following equation.

$$AN=(C1+RRLF)/(C1+RRHF)+C2$$

In the equation given above, AN represents the autonomic nervous system activity, RRHF represents the sum total of the power spectrum of the high-frequency component, and RRLF represents the sum total of the power spectrum of the low-frequency component. Moreover, C1 and C2 are fixed values defined for suppressing divergence of solutions of the autonomic nervous system activity AN.

The activity calculating unit 22 sets an activity threshold value based on the multiple autonomic nerve activities AN calculated for the user, and stores the activity threshold value in the memory 13.

<Guidance method>

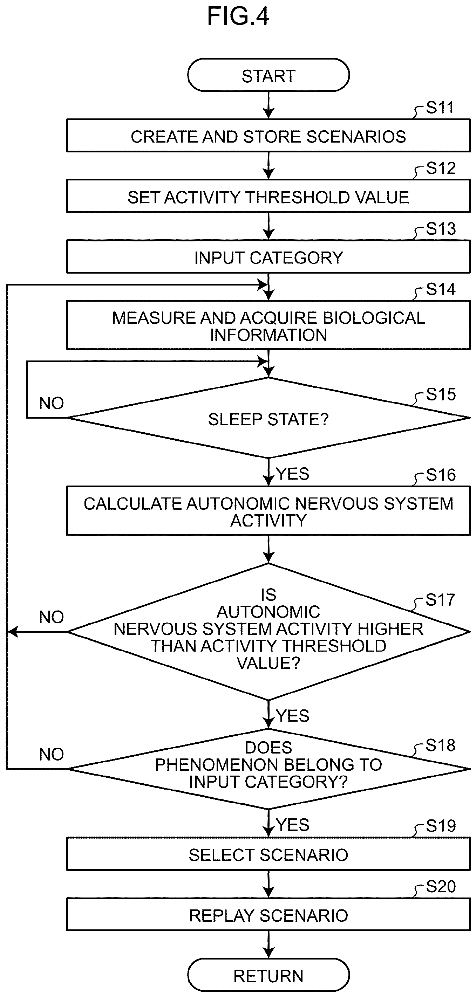

FIG. 4 is a flowchart for explaining a guidance method according to the first embodiment.

As illustrated in FIGS. 1 and 4, at Step S11, the user operates the input unit 11 and creates a scenario. In that case, either the user who uses the guidance device 10 can create a scenario by the input unit 11, or another user other than the user of the guidance device 10 can create a scenario by the input unit 11. Alternatively, scenarios can be created in advance using some other device. The scenario created by operating the input unit 11 is stored in the memory 13. When the scenario is created by another device, the scenario in the another device is stored in the memory 13. At Step S12, the activity threshold value is set. It is desirable that the activity threshold value is set for each user who uses the guidance device 10. The operations at Steps S11 and S12 can be performed before the guidance device 10 is used.

After the operations at Steps S11 and S12 are completed, at Step S13, the user operates the input unit 11 and inputs the category of the dream to be induced. Thus, the user operates the input unit 11 and, from among the categories set in advance, selects the category of the desired dream. Then, the user of the guidance device 10 goes to sleep.

At Step S14, the measuring unit 12 measures the biological information of the user, and the biological information acquiring unit 21 acquires the biological information of the user as measured by the measuring unit 12. At Step S15, based on the biological information of the user, the controller 14 determines whether or not the user is in the sleep state. According to the biological information of the user, when the autonomic nervous system activity calculated using the pulse wave signals is determined to match with the autonomic nervous system activity during the REM sleep or during the non-REM sleep, the controller 14 determines that the user has transitioned into the sleep state. For example, the controller 14 can determine that the user has transitioned into the sleep state based on the brain waves, the pulse waves, the pulse rate, the respiratory rate, and the autonomic nervous system activity as the biological information.

When it is determined that the user is not in the sleep state (No), the controller 14 maintains the present state. On the other hand, when it is determined that the user is in the sleep state (Yes), at Step S16, the activity calculating unit 22 calculates the autonomic nervous system activity based on the biological information of the user acquired by the biological information acquiring unit 21. At Step S17, the determining unit 23 determines whether or not the autonomic nervous system activity, which is calculated by the activity calculating unit 22, is higher than the activity threshold value of the user stored in the memory 13. When the determining unit 23 determines that the autonomic nervous system activity of the user is equal to or lower than the activity threshold value (No), the system control returns to Step S14. Then, a new set of the biological information is acquired, and the operation from Step S15 is performed again.

On the other hand, when the determining unit 23 determines that the autonomic nervous system activity of the user is higher than the activity threshold value (Yes), at Step S18, the determining unit 23 determines whether or not the phenomenon based on the biological information by which the autonomic nervous system activity is determined to be higher than the activity threshold value belongs to the category input by the user. When the determining unit 23 determines that the phenomenon based on the biological information does not belong to the input category (No), the process returns to Step S14. Then, new biological information is acquired, and the processes from Step S15 are performed again. On the other hand, when the determining unit 23 determines that the phenomenon based on the biological information belongs to the input category (Yes), the process proceeds to Step S19.

At Step S19, from among the multiple scenarios stored in the memory 13, the selecting unit 24 selects a scenario related to the biological information of the user acquired by the biological information acquiring unit 21. Thus, based on the biological information by which the autonomic nervous system activity is determined to be higher than the activity threshold value, the selecting unit 24 selects a scenario related to the phenomenon in the dream of the user in the sleep state. At Step S30, the output unit 15 stimulates the brain of the user based on the scenario selected by the selecting unit 24. That is, the output unit 15 stimulates the brain of the user in such a way that multiple pieces of related image data set in the selected scenario is replayed at regular time intervals (for example, intervals of one minute). In that case, the multiple pieces of image data of the phenomenon related to the initially-recalled phenomenon are input in a chronologically manner, and thus the user is supported to have a dream that follows the specific scenario.

Meanwhile, at S30, when the output unit 15 stimulates the brain of the user for providing multiple pieces of image data in a chronological manner based on the scenario and the replay of all pieces of image data is completed, the controller 14 can stimulate the brain for providing the multiple pieces of image data in a chronological manner based on the same scenario again. In that case, based on the biological information, the controller 14 can determine whether the user is in REM sleep state or non-REM sleep state and, when it is determined that the user is in REM sleep state, the controller 14 can stimulate the brain for providing the multiple pieces of image data in a chronological manner based on the same scenario again. When the replay of all pieces of image data is completed by the output unit 15, the processes from Step S14 to Step S30 can be repeated and, based on the biological information of the user, the brain stimulation can be performed for providing the multiple pieces of image data in a chronological manner based on a different scenario related to a newly-recalled phenomenon.

[Second embodiment]

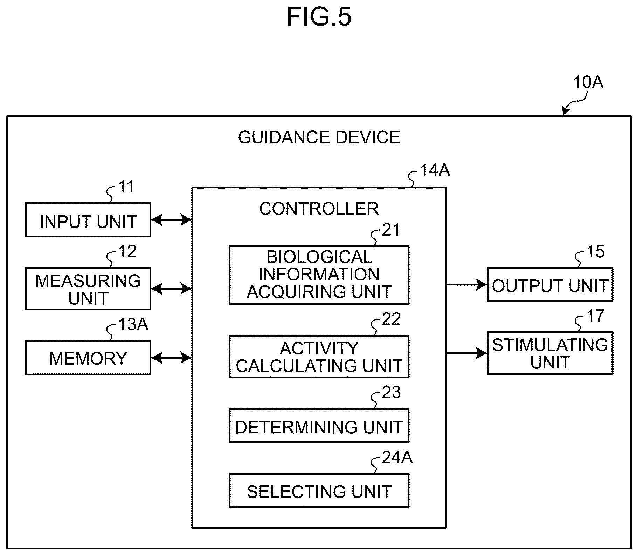

FIG. 5 is a block configuration diagram illustrating a guidance device according to a second embodiment. The constituent elements having identical functions to the functions according to the first embodiment are referred to by the same reference numerals, and their explanation is omitted.

<Guidance device>

As illustrated in FIG. 5, a guidance device 10A includes the input unit 11, the measuring unit 12, a memory 13A, a controller 14A, the output unit 15, and a stimulating unit 17.

The input unit 11, the measuring unit 12, and the output unit 15 are identical to the first embodiment. The memory 13A is connected to the controller 14A. The memory 13A stores therein a variety of information. In the memory 13A, an activity threshold value that is used during a guidance operation performed by the controller 14A is stored in advance. Moreover, in an identical manner to the first embodiment, multiple scenarios are stored in the memory 13A. In addition to storing the multiple scenarios, multiple pieces of tactile information (multiple tactile stimulations) related to the scenarios are also stored in the memory 13A. A tactile stimulation represents one or more stimulations related to a scenario that is created by chronologically arranging multiple pieces of image data associated to each other.

A tactile stimulation is a stimulation applied to skin of the subject, and, for example, is a contact stimulation caused by bringing air, a liquid fluid, or a solid in contact with the subject, with intensity of stimulation and temperature variation. For example, in the scenario about an airplane that is created by chronologically arranging the image data of the first phenomenon (airplane), the image data of the second phenomenon (sky), the image data of the third phenomenon (clouds), the image data of the fourth phenomenon (thunderstorm), the image data of the fifth phenomenon (lightning strike), and the image data of the sixth phenomenon (fire), the tactile stimulation is a contact stimulation applied using the wind that is related to each phenomenon.

The controller 14A includes the biological information acquiring unit 21, the activity calculating unit 22, the determining unit 23, and a selecting unit 24A. The biological information acquiring unit 21, the activity calculating unit 22, and the determining unit 23 are identical to those in the first embodiment.

The selecting unit 24A is connected to the determining unit 23 and the memory 13. Moreover, the selecting unit 24A is connected to the biological information acquiring unit 21, the determining unit 23, and the memory 13A. The selecting unit 24A selects, from among the multiple scenarios stored in the memory 13A, a scenario related to the biological information of the user acquired by the biological information acquiring unit 21. That is, when it is determined that the autonomic nervous system activity calculated by the determining unit 23 based on the biological information of the user is higher than the activity threshold value, the selecting unit 24 selects a scenario related to the phenomenon in the dream of the user in the sleep state.

Moreover, the selecting unit 24A selects, from among multiple tactile stimulations stored in the memory 13A, a tactile stimulation related to the biological information of the user acquired by the biological information acquiring unit 21. That is, when it is determined that the autonomic nervous system activity calculated by the determining unit 23 based on the biological information of the user is higher than the activity threshold value, the selecting unit 24 selects a tactile stimulation related to the selected scenario.

The stimulating unit 17 applies a tactile stimulation to the user. A tactile stimulation is a contact stimulation caused by bringing air, a liquid fluid, or a solid in contact with the subject. For example, the stimulating unit 17 is attached to some part of the body of the user.

<Guidance method>

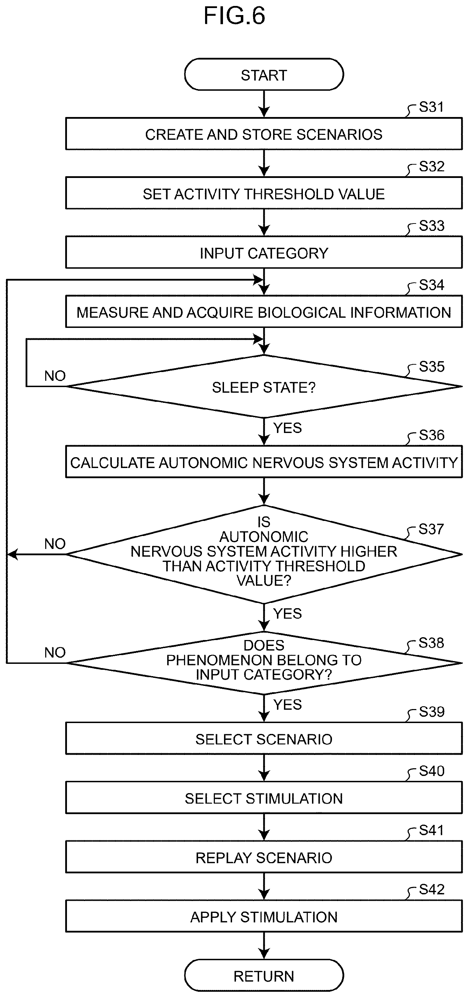

FIG. 6 is a flowchart for explaining a guidance method according to the second embodiment.

As illustrated in FIGS. 5 and 6, at Step S31, the user operates the input unit 11 and creates a scenario. At that time, the scenario created by operating the input unit 11 is stored in the memory 13.

At Step S33, the user operates the input unit 11 and inputs the category of the dream to be induced. Thus, the user operates the input unit 11, and, from the categories set in advance, selects the category of the desired dream. Then, the user of the guidance device 10A goes to sleep.

At Step S34, the measuring unit 12 measures the biological information of the user, and the biological information acquiring unit 21 acquires the biological information of the user measured by the measuring unit 12. At Step S35, based on the biological information of the user, the controller 14A determines whether or not the user is in the sleep state. When it is determined that the user is not in the sleep state (No), the controller 14 maintains the present state. On the other hand, when it is determined that the user is in the sleep state (Yes), at Step S36, the activity calculating unit 22 calculates the autonomic nervous system activity based on the biological information of the user acquired by the biological information acquiring unit 21. At Step S37, the determining unit 23 determines whether or not the autonomic nervous system activity, which is calculated by the activity calculating unit 22, is higher than the activity threshold value of the user stored in the memory 13A. When the determining unit 23 determines that the autonomic nervous system activity of the user is equal to or lower than the activity threshold value (No), the process returns to Step S14. Then, a new set of biological information is acquired, and the processes from Step S35 are performed again.

On the other hand, when the determining unit 23 determines that the autonomic nervous system activity of the user is higher than the activity threshold value (Yes), at Step S38, the determining unit 23 determines whether or not the phenomenon based on the biological information by which the autonomic nervous system activity is determined to be higher than the activity threshold value belongs to the category input by the user. When the determining unit 23 determines that the phenomenon based on the biological information does not belong to the input category (No), the process returns to Step S34. Then, a new set of the biological information is acquired, and the processes from Step S35 are performed again. On the other hand, when the determining unit 23 determines that the phenomenon based on the biological information belongs to the input category (Yes), the process proceeds to Step S39.

At Step S39, from among the multiple scenarios stored in the memory 13, the selecting unit 24A selects a scenario related to the biological information of the user acquired by the biological information acquiring unit 21. At Step S40, the selecting unit 24A selects, from among the multiple tactile stimulations stored in the memory 13, the tactile stimulation related to the biological information of the user acquired by the biological information acquiring unit 21. That is, based on the biological information by which the autonomic nervous system activity is determined to be higher than the activity threshold value, the selecting unit 24A selects the tactile stimulation related to the phenomenon in the dream of the user in the sleep state. More particularly, the selecting unit 24A selects the tactile stimulation related to the selected scenario.

At Step S41, the output unit 15 stimulates the brain of the user based on the scenario selected by the selecting unit 24A. That is, the output unit 15 stimulates the brain of the user in such a way that multiple pieces of related image data set in the selected scenario is replayed at regular time intervals (for example, intervals of one minute). At Step S42, the stimulating unit 17 applies the tactile stimulation to the user based on the tactile stimulation selected by the selecting unit 24A. The multiple pieces of image data of the phenomenon related to the initially-recalled phenomenon are input in a chronologically manner and the tactile stimulation corresponding to the multiple pieces of image data is applied, and thus the user is supported to have a dream that follows the specific scenario.

In the second embodiment, the selecting unit 24A selects the scenario related to the biological information of the user acquired by the biological information acquiring unit 21 and selects the tactile stimulation related to the selected scenario, the output unit 15 stimulates the brain of the user based on the selected scenario, and the stimulating unit 17 applies the tactile stimulation related to the scenario. Alternatively, the selecting unit 24A can select only the tactile stimulation related to the biological information of the user acquired by the biological information acquiring unit 21.

[Effects of embodiments]

The guidance devices according to the embodiments include the biological information acquiring unit 21 that acquires the biological information of the user (subject), the memory 13 or 13A in which multiple scenarios created using multiple of pieces of image data associated to each other are stored, and the selecting units 24 or 24A that select, from among the scenarios stored in the memory 13 or 13A, the scenario related to the biological information acquired by the biological information acquiring unit 21.

Hence, multiple pieces of image data associated to the recalled phenomenon are input in a chronological manner, and the user who is in the sleep state is supported to have a dream that follows the specific scenario. Thus, the user can be guided to have an appropriate dream.

The guidance devices according to the embodiments include the activity calculating unit 22 that calculates the activity of the user based on the biological information acquired by the biological information acquiring unit 21, and the determining unit 23 that compares the activity calculated by the activity calculating unit 22 with a threshold value set in advance. When the determining unit 23 determines that the activity is higher than the threshold value, the selecting units 24 and 24A respectively select the scenario related to the biological information. As a result, it becomes possible to select the dream to be induced.

The guidance devices according to the embodiments include the output unit 15 that stimulates the brain of the user based on the scenarios selected by the corresponding selecting units 24 and 24A. As a result, the user can be guided to have an appropriate dream.

The guidance device according to the second embodiment includes the stimulating unit 17 that applies a tactile stimulation to the user. The tactile stimulations associated to the multiple scenarios are stored in the memory 24a. The selecting unit 24A selects, from the memory 13A, a scenario and a tactile stimulation related to the biological information acquired by the biological information acquiring unit 21. Hence, the multiple pieces of image data of the phenomenon related to the initially-recalled phenomenon are input in a chronologically manner and the tactile stimulation corresponding to the multiple pieces of image data is applied, and thus the user who is in the sleep state is supported to have a dream that follows the specific scenario. Thus, the user can be guided to have an appropriate dream.

In the above, the explanation was given about the guidance device according to the present disclosure. However, the present disclosure can be implemented according to various other forms other than the embodiments described above.

The constituent elements of the guidance device illustrated in the drawings are merely conceptual, and need not be physically configured as illustrated. The constituent elements, as a whole or in part, can be separated or integrated either functionally or physically based on various types of loads or use conditions.

The guidance device is configured using, for example, a program that is loaded as software in a memory. In the embodiment described above, the configuration is explained with reference to function blocks implemented as a result of coordination between hardware and software. Such function blocks can be implemented in various ways, such as using only hardware, or using only software, or using a combination of hardware and software.

Although the present disclosure has been described with respect to a specific embodiment for a complete and clear disclosure, the appended claims are not to be thus limited but are to be construed as embodying all modifications and alternative constructions that may occur to one skilled in the art that fairly fall within the basic teaching herein set forth.

The guidance device, the guidance method, and the program according to the present disclosure can be applied to a technology for controlling the brain activity of the user.

**Reference Signs List**

    10, 10A guidance device
    11 input unit
    12 measuring unit
    13, 13A memory unit
    14, 14A controller

15 output unit
17 stimulating unit
21 biological information acquiring unit
22 activity calculating unit
23 determining unit
24, 24A selecting unit

**Claims**

1. A guidance device comprising:

   a biological information acquiring unit configured to acquire biological information of a subject;
   a memory configured to store therein multiple scenarios created using multiple pieces of image data associated to each other; and
   a selecting unit configured to select, from among the multiple scenarios stored in the memory, the scenario related to the biological information acquired by the biological information acquiring unit.

2. The guidance device according to claim 1, further comprising:

   an activity calculating unit configured to calculate activity of the subject based on the biological information acquired by the biological information acquiring unit; and
   a determining unit configured to compare the activity calculated by the activity calculating unit with a threshold value set in advance, wherein
   the selecting unit is further configured to select, when the determining unit determines that the activity is higher than the threshold value, the scenario related to the biological information.

3. The guidance device according to claim 1 or 2, further comprising an output unit configured to stimulate brain of the subject based on the scenario selected by the selecting unit.

4. The guidance device according to claim 1, further comprising a stimulating unit configured to apply a tactile stimulation to the subject, wherein

   the memory is further configured to store therein tactile stimulations associated to the multiple scenarios, and
   the selecting unit is further configured to select, from the memory, the scenario and the tactile stimulation related to the biological information acquired by the biological information acquiring unit.

5. A guidance method comprising:

   acquiring biological information of a subject;
   storing multiple scenarios created using multiple pieces of image data associated to each other; and
   selecting, from among the stored multiple scenarios, the scenario related to the acquired biological information.

6. A program that causes a computer, which operates as a guidance device, to execute:

   acquiring biological information of a subject;
   storing multiple scenarios created using multiple pieces of image data associated to each other; and
   selecting, from among the stored multiple scenarios, the scenario related to the acquired biological information.

# FIG.1

GUIDANCE DEVICE

10

INPUT UNIT — 11

MEASURING UNIT — 12

MEMORY — 13

CONTROLLER — 14

BIOLOGICAL INFORMATION ACQUIRING UNIT — 21

ACTIVITY CALCULATING UNIT — 22

DETERMINING UNIT — 23

SELECTING UNIT — 24

OUTPUT UNIT — 15

# FIG.2

OUTPUT

R

P

Q S

T

U

W1

TIME

# FIG.3

# FIG.4

START

CREATE AND STORE SCENARIOS — S11

SET ACTIVITY THRESHOLD VALUE — S12

INPUT CATEGORY — S13

MEASURE AND ACQUIRE BIOLOGICAL INFORMATION — S14

SLEEP STATE? — S15
NO

YES

CALCULATE AUTONOMIC NERVOUS SYSTEM ACTIVITY — S16

IS AUTONOMIC NERVOUS SYSTEM ACTIVITY HIGHER THAN ACTIVITY THRESHOLD VALUE? — S17
NO

YES

DOES PHENOMENON BELONG TO INPUT CATEGORY? — S18
NO

YES

SELECT SCENARIO — S19

REPLAY SCENARIO — S20

RETURN

FIG.5

GUIDANCE DEVICE · 10A

INPUT UNIT · 11

MEASURING UNIT · 12

MEMORY · 13A

CONTROLLER · 14A

BIOLOGICAL INFORMATION ACQUIRING UNIT · 21

ACTIVITY CALCULATING UNIT · 22

DETERMINING UNIT · 23

SELECTING UNIT · 24A

OUTPUT UNIT · 15

STIMULATING UNIT · 17

# FIG.6

START

CREATE AND STORE SCENARIOS ⌐S31

SET ACTIVITY THRESHOLD VALUE ⌐S32

INPUT CATEGORY ⌐S33

MEASURE AND ACQUIRE BIOLOGICAL INFORMATION ⌐S34

SLEEP STATE? ⌐S35 — NO

YES

CALCULATE AUTONOMIC NERVOUS SYSTEM ACTIVITY ⌐S36

IS AUTONOMIC NERVOUS SYSTEM ACTIVITY HIGHER THAN ACTIVITY THRESHOLD VALUE? ⌐S37 — NO

YES

DOES PHENOMENON BELONG TO INPUT CATEGORY? ⌐S38 — NO

YES

SELECT SCENARIO ⌐S39

SELECT STIMULATION ⌐S40

REPLAY SCENARIO ⌐S41

APPLY STIMULATION ⌐S42

RETURN

## INTERNATIONAL SEARCH REPORT

| International application No. |
| --- |
| **PCT/JP2024/006217** |

| A. | CLASSIFICATION OF SUBJECT MATTER |
| --- | --- |

*A61M 21/02*(2006.01)i; *A61B 5/16*(2006.01)i
FI:   A61M21/02 F; A61B5/16 110

According to International Patent Classification (IPC) or to both national classification and IPC

| B. | FIELDS SEARCHED |
| --- | --- |

Minimum documentation searched (classification system followed by classification symbols)

A61M21/02; A61B5/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2003-334251 A (DAIKIN INDUSTRIES, LTD.) 25 November 2003 (2003-11-25) entire text, all drawings | 1-6 |
| A | JP 2005-34651 A (HIROSE, Yukihiro) 10 February 2005 (2005-02-10) paragraph [0015] | 1-6 |
| A | US 2014/0221779 A1 (SCHOONOVER, Daniel Carleton) 07 August 2014 (2014-08-07) entire text, all drawings | 1-6 |
| A | CN 1860987 A (BEIJING UNIV) 15 November 2006 (2006-11-15) entire text, all drawings | 1-6 |

☐ Further documents are listed in the continuation of Box C.    ☑ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
| --- | --- |
| **06 March 2024** | **26 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/006217**

| Patent document cited in search report | | | Publication date (day/month/year) | Patent family member(s) | Publication date (day/month/year) |
|---|---|---|---|---|---|
| JP | 2003-334251 | A | 25 November 2003 | (Family: none) | |
| JP | 2005-34651 | A | 10 February 2005 | (Family: none) | |
| US | 2014/0221779 | A1 | 07 August 2014 | (Family: none) | |
| CN | 1860987 | A | 15 November 2006 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5958553 B